# EUROPEAN PATENT APPLICATION

(11) **EP 4 589 293 A1**
(43) Date of publication of application: **23.07.2025**
(21) Application number: 24220454.3
(22) Date of filing: 17.12.2024
(51) Int. Cl.: G01N 33/00, F25D 17/04

(54) **HOUSEHOLD APPLIANCE, ODOR DETECTION METHOD AND SYSTEM FOR HOUSEHOLD APPLIANCE**

(30) Priority: 17.01.2024 CN 202410071324
(71) Applicant: BSH Hausgeräte GmbH, 81739 München (DE)
(72) Inventor: QU, Dean, Nanjing, 210046 (CN)

(57) **Abstract**

A household appliance, and an odor detection method and system for a household appliance are provided. The odor detection method for a household appliance includes: obtaining environmental data in a chamber of the household appliance, where the environmental data includes gas data; and inputting the environmental data into a preset machine learning model and obtaining a classification result, where the preset machine learning model is used to predict a current odor attribute in the chamber based on the environmental data, the preset machine learning model is iteratively updated based on feedback data, and the feedback data is obtained from a user associated with the household appliance and is used to at least represent a predicted satisfaction for the classification result. Through the solution of the present invention, an odor detection logic of the household appliance can be personalized and customized based on a user preference, which is beneficial to improving an intelligence degree of the household appliance.

## Description

### TECHNICAL FIELD

Embodiments of the present invention relate to the technical field of household appliances, and in particular, to a household appliance, and an odor detection method and system for a household appliance.

### BACKGROUND

As one of five senses, the sense of smell has an impact on all aspects of people's daily life and work. For example, an odor emitted in a household appliance during use intuitively affects user experience, which is also one of the evaluation indexes for a user to choose a household appliance.

For example, refrigerators extend the shelf life of foods by forming a low-temperature enclosed space (which may be referred to as a chamber). Therefore, people usually use refrigerators to store perishable foods such as meat and vegetables. Foods placed in refrigerators inevitably generate an unpleasant odor. Because the chamber for storing the foods is enclosed, the unpleasant odor generated by the foods cannot be dispersed by itself, and the unpleasant odor becomes increasingly stronger with the accumulation of time. If the unpleasant odor in the chamber cannot be detected and removed in time, the user experience is adversely affected.

There are mainly two existing solutions for detecting odors in refrigerators: The first is to detect a gas concentration inside the chamber, with the disadvantage that odor types cannot be identified, and therefore odors that cause discomfort to users cannot be removed in a targeted manner, and the second is to detect specific odor types pre-defined in a laboratory, but it is far insufficient to perform odor detection only based on the specific odor types defined in the laboratory. Preferences of different users for odors are different and unpredictable. It is possible that the laboratory defines an odor that is unpleasant, a user A agrees that the odor is unpleasant, but a user B considers that the odor is pleasant. In this case, the same set of odor detection logic cannot satisfy preferences of all users. In addition, sometimes the preferences of the users for the same odor changes over time, but the odor detection logic pre-defined in the laboratory is fixed and unchanged.

Therefore, the odor detection logic of existing household appliances is not intelligent enough to meet individual requirements of users.

### SUMMARY

An objective of embodiments of the present invention is to provide an improved odor detection method for a household appliance. Therefore, an embodiment of the present invention provides an odor detection method for a household appliance, including: obtaining environmental data in a chamber of the household appliance, where the environmental data includes gas data; and inputting the environmental data into a preset machine learning model and obtaining a classification result, where the preset machine learning model is used to predict a current odor attribute in the chamber based on the environmental data, the preset machine learning model is iteratively updated based on feedback data, and the feedback data is obtained from a user associated with the household appliance and is used to at least represent a predicted satisfaction for the classification result.

In the prior art, when odor detection is performed, the same set of detection logic is applied to household appliances of all users, which cannot reflect a personal preference of the user, and is not personalized and intelligent enough. In contrast, the preset machine learning model provided in this implementation solution can be iteratively updated based on feedback of the user, so that the odor detection logic of the household appliance can better match the personal preference of the user using the household appliance. Therefore, the user is allowed to personalize the odor detection logic in the household appliance. The household appliance to which this implementation solution is applied has a higher intelligence degree, and can perform odor detection based on the same preference as the user, so that an odor detection result satisfies the user preference.

Optionally, an iterative update process of the preset machine learning model based on feedback data includes: receiving the feedback data; determining corresponding environmental data based on a generation time of the feedback data; constructing a training set and a verification set based on at least the feedback data and the corresponding environmental data received within a period of time; training the preset machine learning model based on the training set, to obtain an updated preset machine learning model; and verifying the updated preset machine learning model based on the verification set. Therefore, the preset machine learning model is iteratively updated based on the feedback of the user and the environmental data corresponding to current feedback of the user, so that a prediction result of the preset machine learning model more satisfies the user preference, to implement more personalized and intelligent odor detection of the household appliance.

Optionally, the verification set further includes a basic data set, where the basic data set includes standard environmental data and a corresponding standard classification result. Therefore, it is ensured that the prediction result of the preset machine learning model always satisfies basic evaluation criterion, and over-fitting and prediction distortion are avoided. For example, an odor of rotten food is generally considered unpleasant, and the odor of the rotten food is used as content of the basic data set, to ensure that an iteratively updated preset machine learning model always maintains a certain baseline for prediction.

Optionally, the environmental data is periodically collected, and the determining corresponding environmental data based on a generation time of the feedback data includes: determining environmental data whose collection time is closest to the generation time of the feedback data as the corresponding environmental data. Therefore, the feedback data is accurately matched with the environmental data in the chamber of the household appliance that triggers the current feedback of the user, to ensure that the training data provided to the preset machine learning model for training has high accuracy, and ensure that a model training result more satisfies the user preference.

Optionally, the preset machine learning model is periodically and iteratively updated as the feedback data is accumulated. Therefore, the model is continuously trained as usage data of the user is accumulated, prediction of the preset machine learning model is more targeted, to reflect personalization of the user. Further, the preset machine learning model is periodically updated, and a sufficient amount of feedback data is accumulated in each period before an iterative update is uniformly performed, to ensure a better update effect of the preset machine learning model when costs are reduced.

Optionally, the preset machine learning model is iteratively updated based on an initial machine learning model and based on the feedback data, where the initial machine learning model is trained based on a basic data set, and the basic data set includes standard environmental data and a corresponding standard classification result. The initial machine learning model may be preset when the household appliance leaves the factory, and a machine learning algorithm is automatically updated based on the feedback of the user for the odor in the chamber, to obtain a continuously and iteratively updated preset machine learning model, to reflect personalization and intelligence of the household appliance. Further, the same initial machine learning model is preset for each of a plurality of household appliances. As a plurality of users use the household appliances, the preset machine learning model of each household appliance is continuously and iteratively updated based on feedback of each associated user, to increasingly reflect a personal preference of each associated user, so that a difference of prediction results of the preset machine learning models for the same environmental data may be increasingly large. Therefore, personalized odor detection of the user is implemented.

Optionally, the initial machine learning model includes a plurality of sub-models, different sub-models are associated with different odor preferences, and the preset machine learning model being iteratively updated based on the initial machine learning model and based on the feedback data includes: obtaining an odor preference of the user for at least one odor; determining a corresponding sub-model based on the obtained odor preference; and iteratively updating the preset machine learning model based on the determined sub-model and based on the feedback data. Therefore, the user can initially obtain an odor detection solution with a specific personalized function by using the household appliance, and a personalized effect is gradually enhanced as the user uses the odor detection solution. Further, the user uploads a personal preference option, which is recorded and analyzed in the background and connected to the initial machine learning model. A refrigerator of the user with the same or similar personal preference initially uses the same initial machine learning model, and then is gradually updated to a more personalized preset machine learning model as a usage time increases.

Optionally, the method further includes: regularly sending prompt information, where the prompt information includes an opinion feedback form; receiving the opinion feedback form, and generating the feedback data based on the opinion feedback form. Therefore, the user is actively prompted regularly to submit feedback, to collect as much feedback data as possible for training the preset machine learning model, to optimize a model training effect.

Optionally, the prompt information and/or the opinion feedback form is transmitted through a display and/or input unit of the household appliance, and/or the prompt information and/or the opinion feedback form is transmitted through a terminal device associated with the household appliance. Therefore, a human-computer interaction interface arranged on the household appliance prompts the user to perform feedback and receive the feedback data as a basis for model training. Based on the terminal device, it is convenient for the user to receive the opinion feedback form and upload the feedback data anytime and anywhere.

Another objective of the embodiments of the present invention is to provide an improved household appliance.

Therefore, an embodiment of the present invention provides a household appliance, including: a body, defining a chamber; an odor detector, arranged in the chamber to collect environmental data in the chamber; a control module, arranged on the body, where the control module communicates with the odor detector to receive the environmental data, and the control module is configured to perform the foregoing method and generate a control instruction based on a current odor attribute in the chamber; and an odor removal module, arranged in the chamber, where in response to receiving the control instruction, the odor removal module adjusts an operation parameter based on the control instruction.

Therefore, based on the odor detection method provided in this implementation solution, odor detection can be performed based on a personal preference of a user, the odor that is not liked by the user can be removed in time when detected, and the odor in the chamber can be intelligently maintained to satisfy the user preference. Further, actions of model iterative update are all completed in the household appliance, so that the household appliance can complete model optimization without being supported by an external server and without relying on a network, and a response speed is high.

Optionally, the household appliance is selected from a refrigeration appliance, a washing machine, a dishwasher, and an oven.

Optionally, the household appliance further includes a communication module, arranged on the body, where the control module receives feedback data through the communication module; and/or a display and/or input unit, arranged on the body, where the control module receives feedback data through the display and/or input unit. Therefore, the user can transmit the feedback data through network communication, or can directly input the feedback data through the display and/or input unit, to improve convenience of feedback.

Another objective of the embodiments of the present invention is to provide an improved odor detection system for a household appliance.

Therefore, an embodiment of the present invention provides an odor detection system for a household appliance, including a household appliance, including a body and a control module, where the control module is configured to perform the foregoing method; and a server, communicating with the control module, where the server is configured to synchronize a preset machine learning model to the control module.

Therefore, the server may be configured to store historical data, including historically collected environmental data and corresponding feedback data, and retrain the preset machine learning model based on the historical data, so that a current odor attribute subsequently predicted by the household appliance based on an updated preset machine learning model more satisfies a user preference. Further, the server is peripheral to the household appliance, which is beneficial to reducing a quantity of components in the household appliance and reducing costs.

Optionally, the server is configured to iteratively update the preset machine learning model based on feedback data, and synchronize an updated preset machine learning model to the control module. Therefore, with the support of high computing power of the server, an iterative response speed of the preset machine learning model can be further improved.

Optionally, the system further includes a communication module, arranged on the body, where the control module communicates with the server through the communication module. Therefore, the household appliance establishes a communication connection to an outer side through the communication module, to implement remote updating of the preset machine learning model. The outer side may be, for example, the server.

Optionally, the system further includes a display and/or input unit, arranged on the body and communicating with the control module, where the display and/or input unit is configured to receive feedback data. Therefore, the user may locally submit the feedback data at the household appliance, and the household appliance summarizes the feedback data and the corresponding environmental data together to the server, so that the server iteratively updates the preset machine learning model based on these data.

### BRIEF DESCRIPTION OF THE DRAWINGS

FIG. 1 is a flowchart of an odor detection method for a household appliance according to an embodiment of the present invention;
FIG. 2 is a flowchart of an iterative update process of a preset machine learning model based on feedback data according to an embodiment of the present invention;
FIG. 3 is a schematic diagram of a typical application scenario of a household appliance according to an embodiment of the present invention; and
FIG. 4 is a schematic diagram of a principle of an odor detection system for a household
appliance according to an embodiment of the present invention.

In the accompanying drawings:
1-household appliance; 10-body; 101-chamber; 102-refrigerator compartment; 103-freezer compartment; 104-door; 11-odor detector; 12-control module; 13-odor removal module; 14-communication module; 15-display and/or input unit; and 2-server.

### DETAILED DESCRIPTION

To make the foregoing objectives, features, and advantages of the present invention more comprehensible, specific embodiments of the present invention are described in detail below with reference to the accompanying drawings.

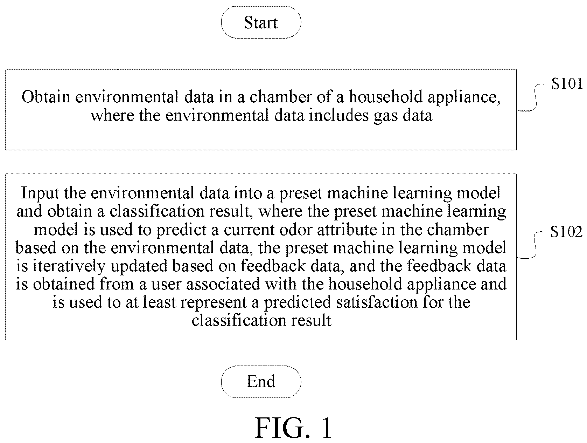

FIG. 1 is a flowchart of an odor detection method for a household appliance according to an embodiment of the present invention.

This implementation solution may be applied to a scenario of detecting an internal odor of a household appliance, for example, detecting whether there is an unpleasant odor inside the household appliance, or detecting a real-time odor attribute in the household appliance. The odor attribute may be used to describe odor pleasantness in the household appliance. According to this implementation solution, odors emitted in the household appliance may be classified, for example, including at least two categories: a pleasant odor and an unpleasant odor. In an actual application, a classification standard of the odor attribute and a quantity of classified categories may be adjusted as needed. For example, a classification result obtained through this implementation solution may be further sub-classified into five categories: very pleasant smell, pleasant smell, neutral smell, unpleasant smell, and extremely unpleasant smell (which may also be referred to as very unpleasant smell).

Items stored in the household appliance may belong to a single type or a plurality of types. In this implementation solution, an odor attribute of an odor generated by a specific type of items in the household appliance may be specifically detected, or an odor attribute of a mixed odor generated by a plurality of items in the household appliance may be entirely detected on a macro scale.

The household appliance in this implementation solution may be selected from a refrigeration appliance, a washing machine, a dishwasher, and an oven.

In an application scenario of odor detection of the refrigeration appliance, an odor of foods stored in a chamber (for example, a refrigerator compartment) of the refrigeration appliance may be detected.

In an application scenario of odor detection of the washing machine, an odor in a washing container (for example, a drum) of the washing machine may be detected. For example, a user does not take out laundry in time after using the washing machine to wash the laundry. Consequently, after the wet laundry remains in the drum for a long time, an odor that causes discomfort to the user may be generated. In this case, this implementation solution may be performed to accurately detect the odor in time.

In an application scenario of odor detection of the dishwasher/oven, an odor in a chamber after the dishwasher/oven is used may be detected. For example, whether odors of cooking, food residues, and the like remain in the dishwasher/oven may be detected through this implementation solution.

This implementation solution may be performed by a control module. The control module may be, for example, a microcontroller of the household appliance, or may be, for example, a control unit in the household appliance dedicated to performing this implementation solution. In this embodiment, the control module may be arranged in a body of the household appliance.

Specifically, referring to FIG. 1, the odor detection method for a household appliance according to this embodiment may include the following steps.

Step S101: Obtain environmental data in a chamber of the household appliance, where the environmental data includes gas data.

Step S102: Input the environmental data into a preset machine learning model and obtain a classification result, where the preset machine learning model is used to predict a current odor attribute in the chamber based on the environmental data, the preset machine learning model is iteratively updated based on feedback data, and the feedback data is obtained from a user associated with the household appliance and is used to at least represent a predicted satisfaction for the classification result.

Further, the gas data may include concentration information of at least one gas component in the chamber. Different types of items may generate different types of gas components in different odor attributes, and intuitive feeling for the user is smelling different odors and generating different levels of evaluation of the odor pleasantness. For example, ingredients may include meats, nuts, vegetables, and the like based on types. The odor attribute may include very pleasant smell, pleasant smell, neutral smell, unpleasant smell, and extremely unpleasant smell.

An evaluation of each odor attribute may be determined based on sensitivity of the user to the odor, or may be determined according to a related industry standard. Further, in this implementation solution, an evaluation criterion of the preset machine learning model during prediction may be corrected in real time based on feedback of the user, so that the classification result more satisfies a personal preference of the user.

In a specific implementation, the gas data may be collected through a gas detector arranged in the chamber. Specifically, step S101 may include the following steps: adjusting an operating temperature of the gas detector during operation of the gas detector, and receiving candidate gas data respectively collected by the gas detector at a plurality of operating temperatures, where the candidate gas data associated with different operating temperatures is used to represent concentrations of different types of gas components and generate the gas data based on a plurality of pieces of received candidate gas data. Therefore, the operating temperature of the gas detector is adjusted in a specific temperature range, to enrich diversity of output data of the gas detector, so that a plurality of gas components is detected.

Further, the gas detector may include a volatile organic compound (VOC) sensor. The VOC sensor reacts violently to a specific type of gas at different temperatures. Based on this characteristic, the gas detector used in this implementation solution can detect the plurality of gas components in a specific temperature range. For example, at a specific operating temperature, the VOC sensor reacts to a specific type of gas component in air, and outputs a resistance value, where the resistance value may be used to represent a concentration of the specific type of gas component.

In some embodiments, the VOC sensor may regularly collect the gas data in the chamber at a preset interval. For example, the VOC sensor collects the gas data in the chamber every n seconds.

Further, each time the gas data is collected, the preset machine learning model outputs the classification result based on the currently collected gas data. Therefore, each time the gas data is collected, a corresponding prediction result of the odor attribute is obtained.

Alternatively, a detection period of the VOC sensor may not be completely consistent with a prediction period of the preset machine learning model. For example, if the VOC sensor collects the gas data in the chamber every 10 seconds, the control module may compare gas data obtained each time, and input newly obtained gas data into the preset machine learning model when detecting that a sudden change in specific values of consecutive two pieces of gas data, to obtain the classification result. In this way, a subsequent prediction operation is performed only when the gas data suddenly changes, which helps reduce computing overheads.

In some embodiments, the environmental data may further include temperature and humidity data in the chamber. Specifically, an environmental temperature and environmental humidity in the chamber may be used as compensation values of the gas data, and are inputted into the preset machine learning model as the environmental data. The three types of data can comprehensively reflect complex gas compositions in the chamber, which is beneficial to improving accuracy of model prediction.

For example, odor detection of the refrigeration appliance is used. In this example, the environmental data may include the gas data and an operation parameter of the refrigeration appliance when the gas data is obtained. The operation parameter may include a compartment temperature and compartment humidity. Further, the operation parameter may be directly obtained from the control module of the refrigeration appliance. In this way, the environmental data required for the model prediction may also be obtained, and the operation parameter is a fixed value and is not affected by an external environment as a door of the refrigeration appliance is opened and closed. Therefore, an effect of using the operation parameter as one piece of the environmental data in improving reliability of input data may be expected.

In a specific implementation, the preset machine learning model may be constructed based on a machine learning algorithm. The machine learning algorithm may be, for example, a decision tree, naive Bayes classification, least squares regression, logistic regression (logistic regression), a support vector machine, a neural network, deep learning, a multilayer perceptron (MLP), a random forest algorithm, an extreme gradient boosting decision tree (XGBoost), and a K-Nearest neighbor (KNN) classification algorithm.

In some embodiments, different odor attributes may be represented by scores of different values or different intervals, and the scores represent levels of the odor pleasantness in the household appliance. For example, a score of 1 indicates pleasant smell, and a score of 0 indicates unpleasant smell. For another example, if a score falls within the range of [0, 1), it indicates unpleasant smell, and if a score is greater than or equal to 1, it indicates pleasant smell.

In step S 102, the output of the preset machine learning model may include a score obtained by scoring the odor pleasantness in the chamber based on the environmental data. A current odor attribute in the chamber may be determined based on the score.

In a specific implementation, in response to obtaining the classification result, the control module may control a corresponding component of the household appliance to perform different actions based on the classification result, to maintain the odor in the chamber at good odor pleasantness. For example, if the classification result is unpleasant smell, the control module may control an odor removal module of the refrigeration appliance to operate to remove the odor.

In a specific implementation, the user associated with the household appliance may be, for example, a user of the household appliance. When using the household appliance, the user may have a subjective determining on the odor emitted by the chamber of the household appliance, and the user feeds back the subjective determining to the household appliance. Based on the feedback of the user, whether the user is satisfied with the odor in the chamber that the user smells may be determined, and then whether the user is satisfied with an operation result of performing a corresponding operation by the control module based on the classification result of the preset machine learning model may be determined. This may essentially represent whether the user is satisfied with the classification result predicted by the preset machine learning model.

For example, assuming that the preset machine learning model predicts that the odor in the chamber is pleasant at a moment, the control module controls the odor removal module to remain in a standby state, but the user feels the odor in the chamber unpleasant when opening the door. In this example, the user may provide the feedback data to feed back that the user is unsatisfied with the odor in the chamber, and the feedback data may indicate that the user is unsatisfied with the classification result predicted by the preset machine learning model.

For another example, assuming that the preset machine learning model predicts that the odor in the chamber is unpleasant at a moment, the control module controls the odor removal module to operate, and the user feels the odor in the chamber pleasant when opening the door. In this example, the user may also provide the feedback data to feed back that the user is satisfied with the odor in the chamber. The feedback data may indicate that the user is satisfied with the classification result of the preset machine learning model.

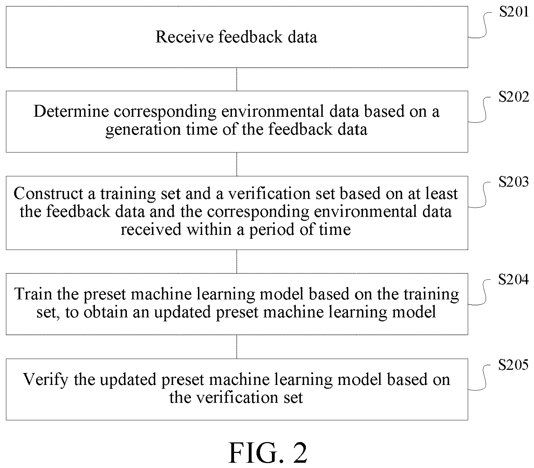

In a specific implementation, referring to FIG. 2, an iterative update process of a preset machine learning model based on feedback data may include the following steps.

Step S201: Receive the feedback data.

Step S202: Determine corresponding environmental data based on a generation time of the feedback data.

Step S203: Construct a training set and a verification set based on at least the feedback data and the corresponding environmental data received within a period of time.

Step S204: Train the preset machine learning model based on the training set, to obtain an updated preset machine learning model.

Step S205: Verify the updated preset machine learning model based on the verification set. Specifically, the feedback data may be received from a user input.

Further, the generation time of the feedback data may be a time at which the user enters the feedback data, or may be a time at which the control module receives the feedback data.

Further, the received feedback data and the corresponding environmental data may be accumulated within a period of time, and the preset machine learning model may be updated after enough data is accumulated.

Therefore, the preset machine learning model is iteratively updated based on the feedback of the user and the environmental data corresponding to current feedback of the user, so that a prediction result of the updated model more satisfies the user preference, to implement more personalized and intelligent odor detection of the household appliance.

In a specific implementation, the environmental data may be periodically collected, and step S202 may include the following step: determining environmental data whose collection time is closest to the generation time of the feedback data as the corresponding environmental data.

Specifically, the periodically collected environmental data may be stored in a storage module. The storage module may be arranged in the household appliance, or may be peripheral to the household appliance and communicate with the control module.

Further, during storage, the environmental data and the collection time of the environmental data may be stored correspondingly. The collection time may be, for example, a timestamp.

In response to receiving the feedback data, the storage module may be searched for the environmental data whose collection time is closest to the generation time based on the generation time of the feedback data, and the environmental data obtained through search is determined as corresponding to the feedback data received this time.

Therefore, the feedback data is accurately matched with the environmental data in the chamber of the household appliance that triggers the current feedback of the user, to ensure that the training data provided to the preset machine learning model for training has high accuracy, and ensure that a model training result more satisfies the user preference.

In some embodiments, a proximity between the collection time of the environmental data and the generation time of the feedback data may be a concept of an absolute value. In other words, the environmental data whose collection time is earlier than or later than the generation time of the feedback data may be considered as the closest environmental data. The control module may select environmental data in which an absolute value of a difference between the collection time and the generation time is the smallest, or use both pieces of environmental data for model training.

In a specific implementation, the training set may be used to train and generate an updated preset machine learning model, and then the verification set is used to verify whether parameters of the updated preset machine learning model are appropriate.

Further, the feedback data and the corresponding environmental data received within a period of time may be divided based on a preset ratio, to obtain the training set and the verification set. For example, the preset ratio may be 7:3, the former is the training set, and the latter is the verification set.

Further, if the verification result in step S205 indicates that accuracy of the prediction result of the updated preset machine learning model is higher than a preset threshold, it may be determined that the updated preset machine learning model satisfies requirements (for example, no over-fitting occurs). In this case, the updated preset machine learning model may be used in a subsequent odor detection solution, that is, the preset machine learning model used in step S 102 may be the updated preset machine learning model described in this example.

The preset threshold may be, for example, 95%. In an actual application, a specific value of the preset threshold may be adjusted based on a user requirement.

In a variant example, if the verification result of step S205 indicates that the accuracy of the prediction result of the updated preset machine learning model is lower than the preset threshold, step S204 and step S205 may be performed again to retrain the preset machine learning model until the updated preset machine learning model passes verification of the verification set.

Further, when step S204 and step S205 are performed again, re-division may be performed to obtain an updated training set and an updated verification set.

In a specific implementation, the verification set may further include a basic data set, where the basic data set includes standard environmental data and a corresponding standard classification result.

Specifically, the basic data set may include laboratory data, and may be specifically a standard result that is manually pre-calibrated. For an odor emitted by a specific type of item, people usually have consistent judgments on the odor. Therefore, environmental data and a corresponding classification result of the odor are defined in a laboratory pre-calibrated manner as standard environmental data and a corresponding standard classification result.

For example, an odor attribute of rotten meat is usually unpleasant, and environmental data when the rotten meat is stored in the chamber may be collected as the standard environmental data, and is correspondingly stored in the basic data set with a corresponding standard classification result "unpleasant".

Further, in step S203, a part of the feedback data and the corresponding environmental data received within a period of time may be grouped into the training set, and a remaining part of the basic data set and the feedback data and the corresponding environmental data received within a period of time may be jointly grouped into the verification set.

Therefore, it is ensured that the prediction result of the preset machine learning model always satisfies basic evaluation criterion, and over-fitting and prediction distortion are avoided. For example, an odor of rotten food is generally considered unpleasant, and the odor of the rotten food is used as content of the basic data set, to ensure that an iteratively updated preset machine learning model always maintains a certain baseline for prediction.

In a specific implementation, the preset machine learning model may be periodically and iteratively updated as the feedback data is accumulated.

Specifically, an update period of the preset machine learning model may be a fixed time interval, such as every day or every week.

Alternatively, the update period of the preset machine learning model may be dynamically adjusted based on an accumulation of the feedback data, and an iterative update is triggered once each time a sufficient amount of feedback data is accumulated. For example, an iterative update of the preset machine learning model is triggered once every 10 pieces of feedback data are accumulatively received.

Therefore, the model is continuously trained as usage data of the user is accumulated, prediction of the preset machine learning model is more targeted, to reflect personalization of the user. Further, the preset machine learning model is periodically updated, and a sufficient amount of feedback data is accumulated in each period before an iterative update is uniformly performed, to ensure a better update effect of the preset machine learning model when costs are reduced.

In a specific implementation, the preset machine learning model may be iteratively updated based on an initial machine learning model and based on the feedback data, where the initial machine learning model is trained based on the basic data set.

Specifically, the initial machine learning model may be preset when the household appliance leaves the factory. As the user uses the household appliance, the environmental data and the corresponding feedback data are continuously accumulated, and the initial machine learning model continuously and iteratively updates the preset machine learning model and the updated preset machine learning model based on the user preference.

For example, the initial machine learning model is generated based on the basic data set, the preset machine learning model is iteratively updated as the feedback data of the user is accumulated, the model is verified through the basic data set and a part of the feedback data of the user, and after the model passes the verification, the household appliance is upgraded for next odor attribute prediction.

Therefore, the machine learning algorithm is automatically updated based on the feedback of the user for the odor in the chamber, to obtain a continuously and iteratively updated preset machine learning model, to reflect personalization and intelligence of the household appliance. Further, the same initial machine learning model is preset for each of a plurality of household appliances. As a plurality of users use the household appliances, the preset machine learning model of each household appliance is continuously and iteratively updated based on feedback of each associated user, to increasingly reflect a personal preference of each associated user, so that a difference of prediction results of the preset machine learning models for the same environmental data may be increasingly large. Therefore, personalized odor detection of the user is implemented.

In a specific implementation, the initial machine learning model may include a plurality of sub-models, and different sub-models are associated with different odor preferences.

Specifically, different users may have different preferences for the same odor. For example, some people feel a durian odor pleasant, and some people feel a coriander odor unpleasant.

In this specific implementation, for a situation in which the same environmental data (representing a specific odor) may correspond to different odor attributes, corresponding sub-models are respectively constructed based on the different odor attributes. Different sub-models may be trained through different basic data sets. The different basic data sets are different in that the same standard environmental data may correspond to different standard classification results. For example, feedback of an odor preference of liking the durian odor and feedback of an odor preference of disliking the durian odor correspond to different sub-models. The standard classification results for the durian odor are pleasant smell and unpleasant smell respectively in the basic data sets of the two sub-models.

Further, the preset machine learning model being iteratively updated based on the initial machine learning model and based on the feedback data may include: obtaining the odor preference of the user for at least one odor; determining a corresponding sub-model based on the obtained odor preference; and iterative updating the preset machine learning model based on the determined sub-model and based on the feedback data.

For example, after the user purchases the household appliance, the odor preference of the user may be obtained in a manner such as a questionnaire. In response to obtaining the odor preference of the user, the control module selects a corresponding sub-model as an actually used initial machine learning model, and then continuously and iteratively updates the initial machine learning model as the user uses the initial machine learning model based on subsequent feedback data of the user.

It is assumed that the user feeds back that the user likes the durian odor, the initial machine learning model includes a sub-model A (constructed by using environmental data and an odor attribute of the durian odor being pleasant as input data) and a sub-model B (constructed by using the environmental data and the odor attribute of the durian odor being unpleasant as input data). In response to receiving feedback data that the user likes the durian odor, the control module may determine that the sub-model A is a sub-model corresponding to the odor preference of the user.

Therefore, the user can initially obtain an odor detection solution with a specific personalized function by using the household appliance, and a personalized effect is gradually enhanced as the user uses the odor detection solution. Further, the user uploads a personal preference option, which is recorded and analyzed in the background and connected to the initial machine learning model. A refrigerator of the user with the same or similar personal preference initially uses the same initial machine learning model, and then is gradually updated to a more personalized preset machine learning model as a usage time increases.

In a specific implementation, for the feedback data and the corresponding environmental data received within a period of time, before the training set and the verification set are constructed, data pre-processing may be first performed on the obtained data.

Specifically, the data pre-processing may include data cleaning, to achieve a denoising effect. For example, abnormal data in the obtained data, such as a peak value in a piece of continuous data, may be removed. For another example, incorrect data, such as data that has a packet loss during transmission, may be removed.

In a specific implementation, the odor detection method according to this implementation solution may further include the following steps: regularly sending prompt information, where the prompt information includes an opinion feedback form; receiving the opinion feedback form, and generating the feedback data based on the opinion feedback form.

Specifically, the prompt information may be sent at a fixed time each day, to collect as much feedback data as possible for training the preset machine learning model, to optimize a model training effect.

Alternatively, the prompt information may be sent each time when it is detected that the user uses the household appliance. For example, after detecting a door opening and closing action, the control module of the refrigeration appliance actively sends the prompt information.

Alternatively, the feedback data may be actively triggered and uploaded by the user.

In a specific implementation, the prompt information and/or the opinion feedback form may be transmitted through a display and/or input unit of the household appliance.

Specifically, the display and/or input unit may include a user interface module (UIM).

For example, a pop-up window may be regularly pops up on a control panel of the household appliance, to query the user whether the user is satisfied with the odor in the current chamber.

Therefore, a human-computer interaction interface arranged on the household appliance prompts the user to perform feedback and receive the feedback data as a basis for model training.

In a specific implementation, the prompt information and/or the opinion feedback form may be transmitted through a terminal device associated with the household appliance. For example, the control module may regularly send the prompt information to the terminal device. The prompt information may include the opinion feedback form, and the terminal device receives the opinion feedback form filled in and submitted by the user. Therefore, the user can receive the opinion feedback form and upload the feedback data anytime and anywhere, so that a feedback process is more convenient.

In some embodiments, the terminal device may communicate with the household appliance, or be controlled by the same user as the household appliance. The terminal device may be, for example, a mobile terminal such as a mobile phone, a tablet PC, or a notebook computer, or may be, for example, another smart household appliance located in the same local area network as the household appliance, such as a washing machine or a cooker hood in the home of the user.

In a specific implementation, the prompt information and the opinion feedback form may be displayed or received through different media. For example, the prompt information may be sent through the display and/or input unit of the household appliance, and the opinion feedback form may be received on the mobile phone of the user.

In conclusion, the preset machine learning model provided in this implementation solution can be iteratively updated based on the feedback of the user, so that the odor detection logic of the household appliance can better match the personal preference of the user using the household appliance. Therefore, the user is allowed to personalize the odor detection logic in the household appliance. The household appliance to which this implementation solution is applied has a higher intelligence degree, and can perform odor detection based on the same preference as the user, so that an odor detection result satisfies the user preference.

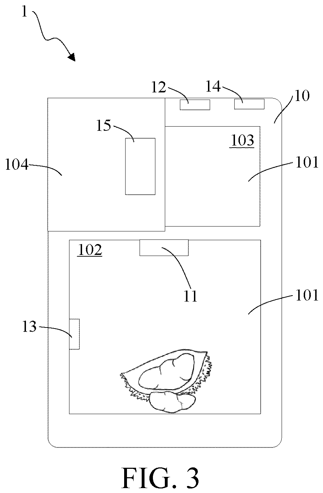

FIG. 3 is a schematic diagram of a typical application scenario of a household appliance 1 according to an embodiment of the present invention. This application scenario is described in detail below by using an example in which the household appliance 1 is a refrigeration appliance. The refrigeration appliance may include a refrigerator, a refrigerated cabinet, and the like.

Specifically, referring to FIG. 3, the household appliance 1 in this embodiment may include a body 10, defining a chamber 101. For example, the chamber 101 of the refrigeration appliance may include a refrigerator compartment 102 and a freezer compartment 103. The chamber 101 may have an opening. The household appliance 1 may include a door 104 to open or close the opening corresponding to the chamber 101.

Further, the household appliance 1 may further include an odor detector 11 arranged in the chamber 101, to collect environmental data in the chamber 101. For example, the odor detector 11 may include a gas detector arranged at the top of the refrigerator compartment 102 and configured to collect gas data in the refrigerator compartment 102. For another example, the gas detector may be arranged near a return air outlet of the refrigeration appliance, to collect overall gas data in the refrigeration appliance. The gas detector may be, for example, a VOC sensor.

Further, the household appliance 1 may further include a control module 12 arranged in the body 10. The control module 12 communicates with the odor detector 11 to receive the environmental data. The control module 12 may be configured to perform the odor detection method shown in FIG. 1 and FIG. 2 and generate a control instruction based on a current odor attribute in the chamber 101.

The control module 12 may include a main control board/a microcontroller of the refrigeration appliance. Alternatively, the control module 12 may be a module dedicated to performing the method shown in FIG. 1 and FIG. 2.

Further, the household appliance 1 may further include an odor removal module 13 arranged in the chamber 101, and in response to receiving the control instruction, the odor removal module 13 adjusts an operation parameter based on the control instruction.

The odor removal module 13 and the odor detector 11 may be arranged in the same chamber 101, for example, both are arranged in the refrigerator compartment 102. Alternatively, the odor removal module 13 may be arranged in an air duct of the refrigeration appliance, and in response to the control instruction, the odor removal module 13 operates based on the operation parameter indicated by the control instruction, to implement overall odor removal of all the chambers 101 through a circulating refrigeration system in the refrigeration appliance.

The odor removal module 13 may be, for example, an ionizer.

Communication between the odor detector 11 and the control module 12, and between the control module 12 and the odor removal module 13 may be in a wired or wireless manner.

Therefore, based on the odor detection method provided in this implementation solution, odor detection can be performed based on a personal preference of a user, the odor that is not liked by the user can be removed in time when detected, and the odor in the chamber 101 can be intelligently maintained to satisfy the user preference. Further, actions of model iterative update are all completed in the household appliance 1, so that the household appliance 1 can complete model optimization without being supported by an external server and without relying on a network, and a response speed is high.

In some embodiments, a temperature and humidity sensor (not shown in the figure) may be further arranged in the chamber 101 to collect temperature and humidity data in the chamber 101.

In a specific implementation, the household appliance 1 may further include a storage module (not shown in the figure), configured to store a preset machine learning model. When performing step S102 shown in FIG. 1, the control module may access the storage module to invoke the preset machine learning model.

Further, in response to performing step S201 to step S205 shown in FIG. 2, an updated preset machine learning model verified through a verification set may cover the preset machine learning model originally stored in the storage module, for the control module to invoke the preset machine learning model when performing step S 102 next time.

In a specific implementation, the household appliance 1 may further include a communication module 14 arranged in the body 10, and the control module 12 receives feedback data through the communication module 14. For example, the communication module 14 may communicate with a terminal device of the user, to receive the feedback data sent by the user through the terminal device. In some embodiments, the communication module 14 may communicate with the terminal device in a wireless communication manner such as a wireless fidelity (Wi-Fi) technology or near field communication (NFC).

The control module 12 may send prompt information to the terminal device of the user through the communication module 14, and receive an opinion feedback form filled in by the user.

Further, the control module 12 may communicate with the odor detector 11 through the communication module 14.

Further, the control module 12 and the odor removal module 13 may also communicate with each other through the communication module 14. It should be noted that, FIG. 3 only exemplarily shows possible arrangement positions of the control module 12, the odor removal module 13, the odor detector 11, and the communication module 14 on the household appliance 1. In an actual application, a relative position relationship of the modules and specific arrangement positions of the modules on the household appliance 1 may be adjusted as needed. The modules may be independent of each other, or may be integrated on the same chip or integrated into the same functional module. For example, the control module 12 and the communication module 14 may be integrated together.

In a specific implementation, the household appliance 1 may further include a display and/or input unit 15 arranged in the body 10, and the control module 12 receives the feedback data through the display and/or input unit 15.

Specifically, the household appliance 1 may include an interaction panel, to implement human-computer interaction. The interaction panel may include a touch pad, a display screen, and the like.

The interaction panel may include the display and/or input unit 15. In some embodiments, the display and/or input unit 15 may be a user interface module (UIM), configured to display a setting parameter or a status of the household appliance 1 and/or receive a control instruction inputted by the user.

Further, the interaction panel may further include a cover plate (not shown in the figure) arranged on the front of the display and/or input unit 15, to at least provide protection. The cover plate may be, for example, a front panel of the door 104 of the household appliance 1, or may be, for example, a glass plate that independently covers the front of the UIM. In some embodiments, the display and/or input unit 15 may be close to a rear side of the cover plate, to receive, for example, an input signal inputted by the user by touching the cover plate. The display and/or input unit 15 may include a light emitting member to transmit light through a corresponding region of the cover plate.

In some embodiments, the control module 12 may directly display the prompt information on the display and/or input unit 15, to prompt the user to perform feedback. Further, the control module 12 may receive, through the display and/or input unit 15, the opinion feedback form inputted by the user, to obtain the feedback data.

For example, the display and/or input unit 15 may be a touchscreen, and the user fills in the opinion feedback form through a touch operation.

For another example, the interaction panel may include a gesture sensing region, and the user fills in the opinion feedback form through a gesture operation.

Therefore, the user can transmit the feedback data through network communication, or can directly input the feedback data through the display and/or input unit 15, to improve convenience of feedback.

In a typical application scenario, referring to FIG. 3, it is assumed that the refrigerator compartment 102 has a durian stored therein, environmental data collected by the odor detector 11 in the refrigerator compartment 102 is transmitted to the control module 12, and the control module 12 invokes the preset machine learning model (denoted as a model A) from the storage module, and inputs the obtained environmental data into the preset machine learning model. Assuming that a classification result predicted by the model A is unpleasant smell, the control module 12 controls the odor removal module 13 to operate at first power to remove an unpleasant odor in the refrigerator compartment 102. In addition, the control module 12 stores the obtained environmental data and a collection time of the environmental data.

If the user opens the refrigerator compartment 102, and feels the odor emitted in the refrigerator compartment 102 still unpleasant, the display and/or input unit 15 generates feedback data. The feedback data represents that the user is unsatisfied with the classification result predicted by the model Athis time. In response to receiving the feedback data, the control module 12 stores the feedback data, and records a generation time of the feedback data. In addition, the control module 12 may further determine the environmental data corresponding to the feedback data.

After 10 sets of the feedback data and the corresponding environmental data are accumulated, the control module 12 retrains the model A based on the 10 sets of data and a basic data set. After the updated preset machine learning model passes verification of the verification set (denoted as a model B), the control module 12 stores the model B to the storage module to replace the original model A.

In this application scenario, compared with the model A, a classification result of the model B for the same input data has at least the following difference: for environmental data that is also collected when the durian is stored in the refrigerator compartment 102, the classification result predicted by the model B is extremely unpleasant smell. Compared with a classification result of unpleasant smell outputted by the model A, based on the classification result of extremely unpleasant smell outputted by the model B, the control module 12 may control the odor removal module 13 to operate at second power, where the second power is greater than the first power. Therefore, the preset machine learning model is iteratively updated based on the feedback of the user, so that in a scenario in which the durian is stored in the refrigerator compartment 102, the odor removal module 13 can actively operate with a greater odor removal capability to better remove the unpleasant odor in the refrigerator compartment 102, to improve a satisfaction for the odor when the user opens the refrigerator compartment 102 next time.

In some embodiments, the model A and the model B may use the same machine learning algorithm, with a difference in adjustment of model parameters.

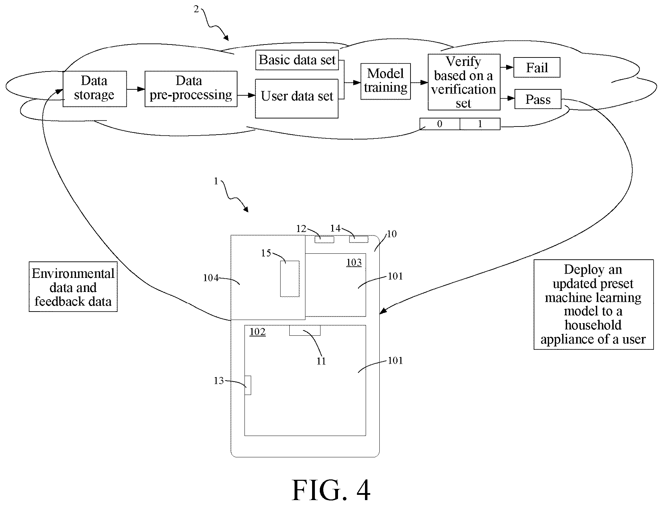

FIG. 4 is a schematic diagram of a principle of an odor detection system for a household appliance according to an embodiment of the present invention.

Specifically, referring to FIG. 4, the odor detection system for a household appliance according to this embodiment may include the household appliance 1 (shown in FIG. 3), including the body 10 and the control module 12. The control module 12 is configured to perform the method shown in FIG. 1 and FIG. 2. A server 2 communicates with the control module 12, and the server 2 is configured to synchronize a preset machine learning model to the control module 12.

Specifically, the preset machine learning model synchronized by the server 2 to the control module 12 may be a preset machine learning model after a last iterative update. For example, if an initial machine learning model is not preset when the household appliance 1 leaves the factory, after being powered on for the first time, the household appliance 1 is connected to the server 2 through the communication module 14, and obtains the initial machine learning model from the server 2. Then, as feedback data of a user is accumulated, the initial machine learning model is continuously and iteratively updated. Each time a version is updated, the server 2 synchronizes an updated preset machine learning model to the control module 12 of the household appliance 1.

In some embodiments, the server 2 may be integrated into the household appliance 1 to be dedicated to dynamically updating a preset machine learning model for the household appliance 1.

In some embodiments, the server 2 may be, for example, a backend server arranged at a manufacturer or designer of the household appliance 1. Alternatively, the server 2 may be, for example, a cloud. A single server 2 may communicate with a plurality of household appliances 1. For each household appliance 1, the server 2 receives environmental data of the household appliance 1 and corresponding feedback data, to dynamically update the preset machine learning model for the household appliance 1 in a targeted manner.

Therefore, the server 2 may be configured to store historical data, including historically collected environmental data and corresponding feedback data, and retrain the preset machine learning model based on the historical data, so that a current odor attribute subsequently predicted by the household appliance 1 based on the updated preset machine learning model more satisfies a user preference.

Further, the server 2 is peripheral to the household appliance 1, which is beneficial to reducing a quantity of components in the household appliance 1 and reducing costs.

In a specific implementation, the server 2 may be configured to iteratively update the preset machine learning model based on the feedback data, and synchronize the updated preset machine learning model to the control module 12. Therefore, with the support of high computing power of the server 2, an iterative response speed of the preset machine learning model can be further improved.

For example, the server 2 may obtain the feedback data from the household appliance 1 or a terminal device of the user, and obtain the corresponding environmental data from the household appliance 1. Further, based on the feedback data and the corresponding environmental data accumulated within a period of time, the server 2 may retrain the preset machine learning model, to implement iterative update of the model.

In a specific implementation, the system in this implementation solution may further include the communication module 14 arranged in the body 10, and the control module 12 communicates with the server 2 through the communication module 14.

For example, the communication module 14 may include a Wi-Fi module, and the control module 12 remotely uploads collected data (such as the environmental data and the feedback data) to the server 2 through the Wi-Fi module.

Further, the Wi-Fi module may be further configured to upgrade the preset machine learning model via an over-the-air (OTA) technology. For example, an OTA platform may be constructed on the server 2, and the Wi-Fi module upgrades the updated preset machine learning model via OTA.

Therefore, the household appliance 1 establishes a communication connection to an outer side through the communication module 14, to implement remote updating of the preset machine learning model. The outer side may be, for example, the server 2.

In a specific implementation, the system in this implementation solution may further include the display and/or input unit 15 arranged on the body 10 and communicates with the control module 12. The display and/or input unit 15 is configured to receive the feedback data. Therefore, the user may locally submit the feedback data at the household appliance 1, and the household appliance 1 summarizes the feedback data and the corresponding environmental data together to the server 2, so that the server 2 iteratively updates the preset machine learning model based on these data.

In a typical application scenario, with reference to FIG. 3 and FIG. 4, an example in which the household appliance 1 is a refrigerator is used. The control module 12 of the refrigerator may perform the method in the embodiments shown in FIG. 1 and FIG. 2, and the refrigerator may include the storage module to store the preset machine learning model.

The odor detector 11 (for example, the VOC sensor) may be arranged in the refrigerator compartment 102 of the refrigerator. The VOC sensor regularly collects the gas data in the refrigerator compartment 102.

In response to receiving the gas data, the control module 12 uploads obtained gas data and a collection time thereof to the server 2. In addition, the control module 12 invokes the preset machine learning model stored in the storage module, to predict a current odor attribute in the refrigerator compartment 102 based on the obtained gas data.

Assuming that a classification result predicted by the preset machine learning model is neutral smell, the control module 12 does not trigger the odor removal module 13 to operate.

During regularly performing the foregoing collection/uploading operation on the gas data, and controlling the odor removal module 13 to operate based on a model prediction result, the control module 12 further regularly sends the prompt information on the display and/or input unit 15 and the terminal device of the user.

If the user opens the refrigerator compartment 102 to feel a strong unpleasant odor in the refrigerator compartment 102, the user fills in the opinion feedback form through an application (APP) installed on the terminal device, and the opinion feedback form may be directly transmitted to the server 2 to generate the feedback data. In response to receiving the feedback data, the server 2 searches for gas data with the closest collection time based on the generation time of the feedback data, and stores these data first correspondingly.

When the feedback data obtained from the user and a data volume of the corresponding gas data are accumulated for a period of time, the server 2 retrains the preset machine learning model based on the accumulated data within this period of time.

Specifically, these data may be pre-processed first. Then, the pre-processed gas data and the corresponding environmental data are used as a user data set with the basic data set as input data to train the preset machine learning model.

If the preset machine learning model passes verification of the verification set, the server 2 synchronously updates the updated preset machine learning model to the refrigerator via the communication module 14 of the refrigerator through the OTA platform.

If the preset machine learning model does not pass verification of the verification set, the model is retrained, or the model is retrained after waiting for a period of time to accumulate more data.

In a variant example, the VOC sensor may include an interaction module, and the user may upload the feedback data through the interaction module.

In a variant example, the feedback data may be uploaded to the server 2 via the communication module 14 through the display and/or input module 15 of the refrigerator.

Although specific implementation solutions are described above, the implementation solutions are not intended to limit the scope of the present invention, even in a case that only a single implementation solution is described with respect to a specific feature. Feature examples provided in the present invention are intended to be illustrative rather than limiting, unless otherwise described. In a specific implementation, technical features of one or more dependent claims may be combined with technical features of independent claims, and technical features from corresponding independent claims may be combined in any proper manner rather than only in a specific combination listed in claims.

Although the present invention is disclosed above, the present invention is not limited thereto. Any person skilled in the art may make various changes and modifications without departing from the spirit and scope of the present invention. Therefore, the protection scope of the present invention should be subject to the scope defined by claims.

## Claims

1. An odor detection method for a household appliance, **characterized by** comprising:
obtaining environmental data in a chamber of the household appliance, wherein the environmental data comprises gas data; and
inputting the environmental data into a preset machine learning model and obtaining a classification result, wherein the preset machine learning model is used to predict a current odor attribute in the chamber based on the environmental data, the preset machine learning model is iteratively updated based on feedback data, and the feedback data is obtained from a user associated with the household appliance and is used to at least represent a predicted satisfaction for the classification result.

2. The method according to claim 1, **characterized in that** an iterative update process of the preset machine learning model based on feedback data comprises:
receiving the feedback data;
determining corresponding environmental data based on a generation time of the feedback data;
constructing a training set and a verification set based on at least the feedback data and the corresponding environmental data received within a period of time;
training the preset machine learning model based on the training set, to obtain an updated preset machine learning model; and
verifying the updated preset machine learning model based on the verification set.

3. The method according to claim 2, **characterized in that** the verification set further comprises a basic data set, wherein the basic data set comprises standard environmental data and a corresponding standard classification result.

4. The method according to claim 2, **characterized in that** the environmental data is periodically collected, and the determining corresponding environmental data based on a generation time of the feedback data comprises:
determining environmental data whose collection time is closest to the generation time of the feedback data as the corresponding environmental data.

5. The method according to claim 1, **characterized in that** the preset machine learning model is periodically and iteratively updated as the feedback data is accumulated.

6. The method according to claim 1, **characterized in that** the preset machine learning model is iteratively updated based on an initial machine learning model and based on the feedback data, wherein the initial machine learning model is trained based on a basic data set, and the basic data set comprises standard environmental data and a corresponding standard classification result.

7. The method according to claim 6, **characterized in that** the initial machine learning model comprises a plurality of sub-models, different sub-models are associated with different odor preferences, and the preset machine learning model being iteratively updated based on the initial machine learning model and based on the feedback data comprises:
obtaining an odor preference of the user for at least one odor;
determining a corresponding sub-model based on the obtained odor preference; and
iteratively updating the preset machine learning model based on the determined sub-model and based on the feedback data.

8. The method according to claim 1, **characterized by** further comprising:
regularly sending prompt information, wherein the prompt information comprises an opinion feedback form; and
receiving the opinion feedback form, and generating the feedback data based on the opinion feedback form.

9. The method according to claim 8, **characterized in that** the prompt information and/or the opinion feedback form is transmitted through a display and/or input unit of the household appliance, and/or the prompt information and/or the opinion feedback form is transmitted through a terminal device associated with the household appliance.

10. A household appliance, **characterized by** comprising:
a body (10), defining a chamber (101);
an odor detector (11), arranged in the chamber (101) to collect environmental data in the chamber (101);
a control module (12), arranged on the body (10), wherein the control module (12) communicates with the odor detector (11) to receive the environmental data, and the control module (12) is configured to perform the method according to any one of claims 1 to 9 and generate a control instruction based on a current odor attribute in the chamber (101); and
an odor removal module (13), arranged in the chamber (101), wherein in response to receiving the control instruction, the odor removal module (13) adjusts an operation parameter based on the control instruction.

11. The household appliance according to claim 10, **characterized in that** the household appliance is selected from a refrigeration appliance, a washing machine, a dishwasher, and an oven.

12. The household appliance according to claim 10, **characterized by** further comprising:
a communication module (14), arranged on the body (10), wherein the control module (12) receives feedback data through the communication module (14); and/or
a display and/or input unit (15), arranged on the body (10), wherein the control module (12) receives feedback data through the display and/or input unit (15).

13. An odor detection system for a household appliance, **characterized by** comprising:
a household appliance, comprising a body (10) and a control module (12), wherein the control module (12) is configured to perform the method according to any one of claims 1 to 9; and
a server (2), communicating with the control module (12), wherein the server (2) is configured to synchronize a preset machine learning model to the control module (12).

14. The system according to claim 13, **characterized in that** the server (2) is configured to iteratively update the preset machine learning model based on feedback data, and synchronize an updated preset machine learning model to the control module (12).

15. The system according to claim 13, **characterized by** further comprising:
a communication module (14), arranged on the body (10), wherein the control module (12) communicates with the server (2) through the communication module (14).

16. The system according to claim 13, **characterized by** further comprising:
a display and/or input unit (15), arranged on the body (10) and communicating with the control module (12), wherein the display and/or input unit (15) is configured to receive feedback data.
